# EUROPÄISCHE PATENTSCHRIFT

(11) **EP 2 247 267 B1**
(45) Veröffentlichungstag und Bekanntmachung des Hinweises auf die Patenterteilung: **04.11.2015**
(21) Anmeldenummer: 09716782.9
(22) Anmeldetag: 06.03.2009
(51) Int. Cl.: A61F 2/78, A61F 2/50

(54) **VERFAHREN ZUM HERSTELLEN EINES ANGEPASSTEN LINERS**
METHOD FOR PRODUCING AN ADAPTED LINER
PROCÉDÉ DE RÉALISATION D'UN MANCHON ADAPTÉ

(30) Priorität: 07.03.2008 DE 102008013527
(43) Veröffentlichungstag der Anmeldung: 10.11.2010
(73) Patentinhaber: Otto Bock HealthCare GmbH, 37115 Duderstadt (DE)
(72) Erfinder: REINELT, Stefan, 37115 Duderstadt (DE)
(74) Vertreter: Stornebel, Kai
(86) Internationale Anmeldenummer: PCT/DE2009/000308
(87) Internationale Veröffentlichungsnummer: WO 2009/109182

(56) Entgegenhaltungen:
- WO-A1-98/04218
- WO-A1-99/45862
- DE-A1- 19 823 753
- DE-U- 1 959 638
- US-A1- 2002 165 619

## Beschreibung

Die Erfindung betrifft ein Verfahren zum Herstellen eines angepassten Liners.

Liner werden in der Prothetik als ein Zwischenelement zwischen dem Körperglied und der Prothese vorgesehen. Der Liner geht eine innige Verbindung zum Stumpf ein, darüber hinaus ist es möglich, dass der Liner über eine mechanische Arretierung mit der Prothese verbunden wird. Darüber hinaus wird über einen Unterdruck ein verbessertes Haften der Prothese an dem Liner gewährleistet, so dass der mit dem Liner versorgte Patient ein verbessertes Prothesengefühl und eine verbesserte Sicherheit gegenüber einem unfreiwilligen Lösen der Prothese hat.

Solche Liner sind in der Regel aus einem Silikonwerkstoff hergestellt. Alternativ können aber auch Polyurethan- oder Block-Copolymerwerkstoffe eingesetzt werden. Um die Liner an die physiologischen Gegebenheiten des Patienten anpassen zu können, wird der Stumpf, an dem der Liner anliegen soll, abgeformt, ein Stumpf positiv aus Gips oder Kunststoff hergestellt und der Liner individuell auf Grundlage des abgeformten Stumpfes angefertigt. Die individuelle Anfertigung eines Liners ist sehr aufwendig und damit sehr teuer. Bei der Versorgung eines Patienten mit einem solcherart hergestellten Liner entstehen hohe Kosten.

Darüber hinaus sind Standardliner erhältlich, die in verschiedenen Größen vorkonfektioniert gefertigt werden. Eine individuelle Anpassung erfolgt nicht, so dass es trotz unterschiedlicher Größen bei den Standardlinern zu Hohlräumen und damit zu Instabilitäten kommen kann.

Die WO 98/04218 A1 beschreibt Liner, bei denen ein Gewebe von einem aushärtenden Gel umgeben wird. Durch verschiedene Ausgestaltungen der einzelnen Gewebeteile kann der so gefertigte Liner an bestimmte Körperteile, an denen er im angelegten Zustand anliegt, angepasst werden. Insbesondere ist es möglich, durch eine unterschiedliche Dicke der Gelschicht bestimmte Stellen zu polstern und so insbesondere den Stellen Rechnung zu tragen, bei denen ansonsten ein Hohlraum zwischen dem Stumpf des Patienten und dem Liner entstehen würde. Eine Individualisierung eines derartigen Liners ist jedoch zeit- und kostenintensiv, da für jeden Liner neue Gewebestücke angefertigt werden müssen, die den individuellen Gegebenheiten des Patienten angepasst sind.

Aus der WO 99/45862 A1 ist ein Verfahren bekannt, eine Teilbrustprothese auszubilden. Dabei wird eine Flüssigkeit in eine Tasche aus zwei aneinander befestigten Filmschichten eingebracht. In die Flüssigkeit wird eine Kapsel mit einem Härter eingeschlossen. Sobald die Kapsel zerbrochen wird, beginnt die Flüssigkeit auszuhärten. Da sie dabei am Körper des Patienten, beispielsweise im BH der Patientin getragen wird, passt sie sich der Körperkontur des Patienten an.

Aufgabe der vorliegenden Erfindung ist es, ein Verfahren zum Herstellen eines Liners und einen Liner bereitzustellen, mit denen die Nachteile des Standes der Technik vermieden werden können. Erfindungsgemäß wird diese Aufgabe durch ein Verfahren mit den Merkmalen des Anspruchs 1 gelöst. Vorteilhafte Ausgestaltungen und Weiterbildungen der Erfindung sind in den abhängigen Ansprüchen beschrieben.

Das erfindungsgemäße Verfahren zum Herstellen eines angepassten Liners zur Anlage an einem Stumpf, insbesondere an einem Amputationsstumpf, sieht vor, dass zunächst ein vorgefertigter Liner, ein sogenannter Standardliner, in Abhängigkeit von der Größe des Stumpfes ausgewählt wird. Die Herstellung eines vorgefertigten Liners erfolgt nach den üblichen Verfahrensschritten, die aus dem Stand der Technik bekannt sind. Der vorgefertigte Liner besteht überwiegend aus einem Elastomer, beispielsweise Silikon, Polyurethan oder einem Copolymer und ist ggf. auf der Außenseite kaschiert oder mit einer Verstärkungseinlage versehen. Nach dem Auswählen des vorgefertigten Liners wird eine Füllmasse auf Bereiche des Stumpfes aufgebracht, die von der Innenkontur des vorgefertigten Liners abweichen. In der Regel sind die Standardliner rotationssymmetrisch aufgebaut und haben ein geschlossenes Ende, so dass sich insgesamt eine Zylinderform oder eine Paraboloidform einstellt. Sofern von der üblichen, runden Innenkontur abweichende Formen an dem Stumpf auftreten, werden diese mit einer Füllmasse ausgeglichen. Nach dem Aufbringen der Füllmasse in oder an den Bereichen des Stumpfes, die von der Innenkontur des vorgefertigten Liners abweichen, tritt die Füllmasse mit der Innenseite des vorgefertigten Liners in Kontakt und verbindet sich mit dem vorgefertigten Liner, so dass ein personalisierter Liner entsteht. Das Verbinden der Füllmasse mit dem vorgefertigten Liner erfolgt bevorzugt über eine mechanische, physikalische Verbindung der Füllmasse mit dem Liner. Der Liner und die Füllmasse gehen dabei eine innige und feste Verbindung ein. Eine chemische Vernetzung zwischen der Füllmasse und dem Liner findet insofern nicht statt, als der Liner ausreagiert ist. Primer erhöhen die Haftung, ohne eine grenzüberschreitende chemische Vernetzung zu bewirken. Alternativ kann eine stoffschlüssige Verbindung zwischen dem vorgefertigten Liner und der Füllmasse erfolgen, wenn die verwendeten Werkstoffe, in der Regel Elastomere, dies zulassen.

Eine Variante der Erfindung sieht vor, dass die Füllmasse zunächst auf den Stumpf aufgetragen wird, vorzugsweise in den Bereichen, in denen Einschnürungen, Vertiefungen oder dergleichen vorhanden und Abweichungen zu der Innenkontur des vorgefertigten Liners zu erwarten sind. Anschließend wird der vorgefertigte Liner über den Stumpf mit der aufgebrachten Füllmasse angelegt, insbesondere aufgerollt, so dass die Innenseite des Liners mit der Füllmasse in Kontakt tritt. Nach dem Anlegen des Standardliners werden die Füllmasse und der vorgefertigte Liner miteinander verbunden, so dass ein individualisierter Liner entsteht, der auf der Basis des vorgefertigten Standardliners auf einfache Art und Weise hergestellt wurde. Diese Variante hat den Vorteil, dass sie einfach auszuführen ist, keine Spezialwerkzeuge benötigt werden und sich die Füllmasse durch das Anlegen oder Abrollen des vorgefertigten Liners auf dem Stumpf selbsttätig verteilt. Der von dem vorgefertigten Liner aufgebrachte Druck verteilt die Füllmasse dergestalt, dass die Unterschiede zwischen der Stumpfkontur und der Innenkontur des vorgefertigten Liners ausgeglichen werden.

Alternativ dazu ist vorgesehen, dass der vorgefertigte Liner angelegt und anschließend die Füllmasse durch den vorgefertigten Liner in die aufzufüllenden Bereiche eingeführt und aufgebracht wird. Diese Variante hat den Vorteil, dass nach dem Anlegen des vorgefertigten Liners durch das in der Regel durchsichtige oder durchscheinende Material des Liners erkannt werden kann, wo der vorgefertigte Liner nicht vollständig an dem Stumpf anliegt, wo sich also Freiräume zwischen dem Stumpf und der Innenkontur des vorgefertigten Liners bilden. In diese Freiräume wird die Füllmasse eingebracht, beispielsweise durch die Wand des vorgefertigten Liners hindurch mittels einer Spritze oder dergleichen oder von dem offenen, proximalen Ende des Liners her, z.B. über eine Kanüle oder einen Schlauch. Diese Variante hat den Vorteil, dass bei der Individualisierung des vorgefertigten Liners gesehen werden kann, wo Freiräume aufzufüllen sind, so dass sehr genau kontrolliert werden kann, wo und wie viel Füllmasse eingebracht wird.

Die Füllmasse wird insbesondere im Bereich von Narbeneinzügen, schiefen Stumpfebenen, Dysmelien, Amelien oder konkaven Stellen aufgetragen, so dass insgesamt eine angenähert symmetrische Außenkontur des mit der Füllmasse versehenen Stumpfes erreicht wird.

Der vorgefertigte Liner ist bevorzugt als ein Silikonliner und die Füllmasse als ein unvernetzter Silikonkautschuk ausgebildet, um neben den elastischen Eigenschaften des Silikonwerkstoffes auch ein hohes Maß an Hautfreundlichkeit und Haftfähigkeit bereitzustellen. Weiterhin können als Linerwerkstoffe andere Elastomere eingesetzt werden, beispielsweise Polyurethan oder Copolymere. Diese Werkstoffe können auch als Werkstoffe für die Füllmasse verwendet werden, so dass der fertige, individualisierte Liner aus im Wesentlichen einem Werkstoff besteht.

Da der angepasste, individualisierte Liner mit den aufgrund der vernetzten und mit dem Standardliner verbundenen Füllmasse nach innen ragenden Vorsprüngen nicht an der Hautoberfläche kleben bleiben soll, wird vor dem Aufbringen der Füllmasse die Hautoberfläche isoliert, beispielsweise durch Aufbringen von Gipsisoliercreme oder Vaseline, insbesondere damit eine eventuell vorhandene Behaarung an dem Stumpf nicht an der Füllmasse hängen bleibt. Sofern keine Behaarung vorliegt und keine empfindlichen Stellen an dem Stumpf vorhanden sind, kann auf eine vorgeschaltete Isolierung verzichtet werden.

Eine bevorzugte Art und Weise des Aufbringens des Liners auf den Stumpf sieht vor, dass der vorgefertigte Liner mit der Innenseite nach außen gekehrt wird, so dass die während des Tragens untere Innenseite des Liners als eine nach außen gestülpte Kappe vor dem Stumpfende positioniert wird. Anschließend wird von dem Stumpfende her der Liner auf den Stumpf aufgerollt, so dass die Füllmasse nach oben gedrückt wird. Durch diese Art und Weise des Aufrollens und Aufbringens des Liners auf den Stumpf wird sichergestellt, dass keine Luft eingeschlossen wird, so dass keine ungewollten Hohlräume entstehen. Diese Art des Anlegens kann auch eingesetzt werden, wenn die Füllmasse erst nach dem Anlegen eingebracht wird.

Um ein Verbinden der Füllmasse mit der Innenseite des Liners zu erleichtern, wird die Innenseite des vorgefertigten Liners vor dem Anlegen entfettet, so dass aufgrund der Fettfreiheit eine gute Verbindung der Füllmasse mit dem Material des Liners, bevorzugt der Silikone, miteinander gewährleistet wird.

Eine Weiterbildung der Erfindung sieht vor, dass die zunächst unvernetzte Füllmasse beispielsweise aus eine Handpistole zügig auf die Hautoberfläche aufgetragen und unmittelbar danach der Liner über die noch nicht vernetzte Füllmasse gerollt wird, damit sich die noch unvernetzte Füllmasse mit dem vorgefertigten Liner stoffschlüssig verbindet. Diese Verbindung kann über ein Ausvulkanisieren der Füllmasse im angelegten Zustand erfolgen, wobei sich die Füllmasse dann mit dem vorgefertigten Liner stoffschlüssig verbindet.

Nach dem Verbinden der Füllmasse mit dem vorgefertigten Liner wird der Liner abgenommen und eventuell vorhandene Grade werden geglättet, um das Tragen des angepassten Liners zu erleichtern. Sofern noch Nacharbeitungsbedarf besteht, ist es vorgesehen, dass Füllmasse erneut auf den Stumpf aufgebracht und der bereits teilweise angepasste Liner endgültig an den Stumpf angepasst wird, indem der im ersten Durchlauf nur teilweise angepasste Liner erneut angelegt und mit der Füllmasse verbunden wird, so dass Freiräume, die nach der ersten Anpassung noch bestanden, aufgefüllt werden. Die beiden vorbeschriebenen Varianten können miteinander gekoppelt werden, so dass im ersten Durchgang zunächst die Füllmasse vor dem Anlegen des vorgefertigten Liners auf den Stumpf aufgetragen und der Liner anschließend angelegt wird. Sollte sich dann ergeben, dass noch Freiräume vorhanden sind, können diese durch Einbringen weiterer Füllmasse durch den Liner oder von der proximalen Öffnung her aufgefüllt werden. Dieses Einbringen durch den Liner kann auch vor dem Abnehmen des Liners nach dem Verbinden mit der zuvor aufgetragenen Füllmasse erfolgen. Nach dem Aufbringen der Füllmasse und dem anschließenden Anlegen des vorgefertigten Liners kann überprüft werden, ob noch Freiräume zwischen dem Liner und der Stumpfoberflächen vorhanden sind; diese Freiräume können dann unmittelbar durch den Liner hindurch mit der Füllmasse aufgefüllt werden. Ebenso ist es möglich, dass zunächst der vorgefertigte Liner angelegt, Füllmasse eingebracht und nach dem Abnehmen des Liners von dem Stumpf auf die Stumpfoberfläche Füllmasse aufgetragen und der bereits teilindividualisierte Liner angelegt wird, z.B. auf dem Stumpf abgerollt wird.

Der Liner besteht aus einem tubusartigen Grundkörper, der an einem Ende offen und an dem gegenüberliegenden Ende geschlossen ausgebildet ist. Der Grundkörper ist bevorzugt ein vorgefertigter Standardliner. An diesem Grundkörper ist nachträglich, also nach dessen Herstellung, zumindest ein Formkörper innenseitig an dem Grundkörper angebracht ist, der einen Freiraum zwischen dem Grundkörper und dem Stumpf zumindest teilweise auffüllt, wobei der Formkörper an dem Grundkörper stoffschlüssig befestigt ist. Während bei den herkömmlichen, individuellen Linern eine Abformung von einem vorher angefertigten Abdruck oder Abguss erfolgt und die Liner in der Regel in einem Tauchverfahren hergestellt werden, kann mit dem erfindungsgemäßen Liner ein herkömmlicher Standardliner verwendet und individualisiert werden, um so an die jeweilige Form des Stumpfes oder der Gliedmaße angepasst zu werden. Die Freiräume sind die Abweichungen zwischen der Innenkontur des Grundkörpers oder Standardliners und der Außenkontur des Stumpfes.

Der Grundkörper kann auch vorflektiert ausgebildet sein, um durch den angepassten Liner im Gelenkbereich einen besseren Sitz zu erreichen, da er besser den anatomischen Gegebenheiten folgt.

Der Grundkörper und der zumindest eine Formkörper sind bevorzugt aus Silikonkautschuk ausgebildet, wobei die stoffschlüssige Befestigung des Formkörpers an dem Grundkörper durch das Anvulkanisieren erfolgen kann. Ebenfalls können der Grundkörper und die Füllmasse aus einem anderen Elastomerwerkstoff hergestellt sein, beispielsweise einem Copolymer oder einem Polyurethanwerkstoff.

Nachfolgend wird ein Ausführungsbeispiel der Erfindung anhand der beigefügten Figuren näher erläutert. Es zeigen:
- Figur 1 -: einen Stumpf und einen Liner nach dem Aufbringen einer Füllmasse; sowie
- Figur 2 -: einen fertig angepassten Liner nach dem Abnehmen von dem Stumpf.

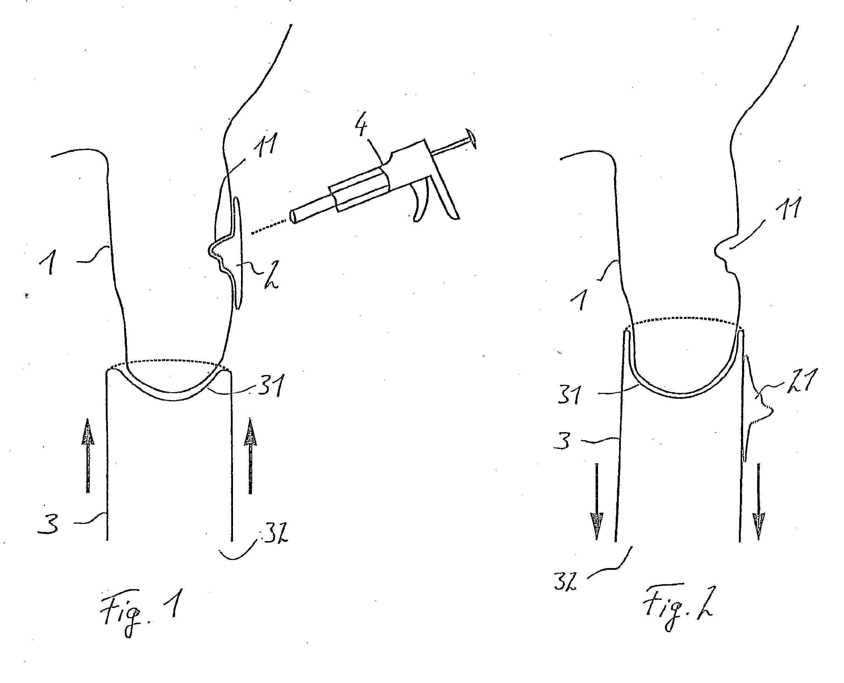

In der Figur 1 ist ein Amputationsstumpf 1 einer Gliedmaße dargestellt. Der Stumpf 1 weist Ungleichmäßigkeiten 11 in seiner Außenform auf, die verschiedene Ursachen haben können. Neben der natürlichen Asymmetrie einer Gliedmaße und der natürlichen Abweichung von einer idealen, glattwandigen Form können die Ungleichmäßigkeiten durch Fehlbildungen, wie Dysmelien oder Amelien, oder aufgrund von Verletzungen oder Operationsnarben herrühren. Dies ist schematisch anhand eines Narbeneinzuges 11 in der Figur 1 dargestellt.

Ebenfalls dargestellt in der Figur 1 ist ein vorbereiteter, vorgefertigter Standardliner 3, dessen eigentliche Innenseite nach außen gekehrt ist. Der Standardliner 3 ist tubusförmig ausgebildet und weist ein geschlossenes Ende 31 und ein gegenüberliegendes offenes Ende 32 auf. In der Ausgangsposition, die durch die gestrichelte Linie dargestellt ist, ergibt sich eine leichte Wölbung nach außen. Das geschlossene Ende 31 wird an das distale Ende des Stumpfes 1 angelegt, so dass sich eine entsprechende, glattwandige, vollflächige Anlage des geschlossenen Endes 31 an dem Stumpf 1 ergibt.

Würde der Standardliner 3 dann weiter in Richtung der Pfeile gemäß Figur 1 nach oben entlang dem Stumpf 1 gezogen werden, würde der Narbeneinzug 11 nicht in Kontakt mit der Linerinnenseite kommen, so dass ein Hohlraum zwischen der Linerinnenseite und der Stumpfoberfläche vorliegen würde, was den Tragekomfort und die Tragesicherheit der anschließend anzulegenden, nicht dargestellten Prothese bedeuten würde.

Daher wird zunächst aus einem Statikmischer 4 eine Füllmasse 2 in den Narbeneinzug 11 als Beispiel für eine ungleichmäßige Ausgestaltung der Stumpfoberfläche aufgebracht. Die Menge der Füllmasse 2 ist dabei so bemessen, dass die Unregelmäßigkeit 11 vollständig ausgeglichen wird, gegebenenfalls kann eine überschüssige Menge aufgetragen und anschließend auf dem umliegenden Stumpf 1 verteilt werden. Das Auftragen erfolgt möglichst blasenfrei und zügig, damit sich die unvernetzte Füllmasse 2 nicht miteinander vernetzen kann. Anschließend wird direkt nach dem Auftragen der Liner 3 als Grundkörper über die noch unvulkanisierte Füllmasse 2 innerhalb der Unregelmäßigkeit 11 und den Stumpf 1 gerollt. Dies erfolgt in Richtung der beiden Pfeile gemäß Figur 1. Durch das Abrollen des Liners 3 von dem distalen Ende des Stumpfes 1 zum proximalen Ende und das enge Anliegen des Liners 3 an dem Stumpf 1 ist es möglich, den Liner 3 ohne Lufteinschluss an den Stumpf 1 anzulegen. Möglicher Materialüberschuss der Füllmasse 2 wird nach oben gedrückt und kann mit einem Spatel abgestrichen werden. Aufgrund der Kompression des Liners 3 auf den Stumpf 1 und die Füllmasse 2 formt sich die Füllmasse 2 automatisch formschlüssig an die Hohlräume, schiefen Ebenen, Dysmelien, Amelien oder Narbeneinzüge 11 an und liegt gleichzeitig an dem Liner 3 an.

Nach der durch das Material vorgegebenen notwendigen Wartezeit hat sich die unvernetzte Silikonkautschukmasse ausvulkanisiert und mit der Innenseite des Liners 3 verbunden. Bevorzugt ist die Füllmasse 2 aus einem unvernetzten Silikonkautschukwerkstoff ausgebildet, der innerhalb kurzer Zeit ausvulkanisiert und sich mit dem ebenfalls aus einem Silikonkautschukwerkstoff hergestellten Grundkörper 3 verbindet.

Nach der stoffschlüssigen Verbindung der Füllmasse 2 mit dem Grundkörper 3 wird der nunmehr angepasste Standardliner 3 von dem Stumpf 1 abgezogen, indem das proximale, offene Ende des Liners 3 in Richtung der Pfeile gemäß Figur 2 in Richtung auf das distale Ende abgezogen wird. Dabei wird die Innenseite des Liners 3 nach außen gekehrt. An dem Liner 3 ist dann ein Formkörper 21 angeformt, der exakt mit der Ausnehmung bzw. der Ungleichmäßigkeit 11 in dem Stumpf 1 korrespondiert. Der angeformte Formkörper 21 kann anschließend bearbeitet werden, beispielsweise mit einer Trichterfräse beschliffen werden, um möglicherweise vorhandene scharfe Kanten zu brechen und um die Oberfläche und Übergänge zu glätten. Sollte es notwendig sein, ein weiteres Mal eine Füllmasse 2 aufzutragen, kann dies auf die gleiche, wie oben beschriebene Art und Weise erfolgen. Mit dieser Technik ist es möglich, Volumendifferenzen zwischen einem Linergrundkörper 3 und einem Stumpf 1 auszugleichen, ohne einen individuellen Abdruck des Stumpfes 1 anfertigen zu müssen. Ebenfalls ist es nicht notwendig, einen Prothesenschaft thermoplastisch an den Stumpf 1 anzuformen oder separate Pads an den Prothesenschaft oder an den Stumpf 1 anzukleben.

Es hat sich überraschend gezeigt, dass sich die Füllmasse aus einem unvernetzten Silikonkautschuk mit den bereits ausvulkanisierten Silikonwerkstoffen des vorgefertigten Liners verbindet. Dabei ist die Verwendung von Haftvermittlern hilfreich, jedoch nicht unbedingt erforderlich. Als Haftvermittler können bei Raumtemperatur vernetzende Silikone eingesetzt werden, die in einer 2-Komponentenzubereitung vorliegen und unmittelbar vor dem Anlegen des vorgefertigten Liners aufgetragen werden können. Als Linermaterial können sowohl hochtemperaturvernetzte als auch niedrigtemperaturvernetzte Silkone eingesetzt werden. Neben bei Raumtemperatur vernetzenden Silikonen, ist der Einsatz von hochtemperaturvernetzenden Silikonen ebenfalls möglich, wobei die hochtemperaturvernetzenden Silikone bei einer Temperatur oberhalb von 100° C zu vulkanisieren beginnen.

Neben Silikonen könne auch andere Elastomere wie Polyurethan oder Copolymere als Werkstoffe für die Füllmasse oder den Grundkörper eingesetzt werden. Neben dem anhand der Figuren beschriebenen Verfahren kann die Füllmasse auch durch den Grundkörper hindurch, also durch ein Loch in der Wand des Grundkörpers eingebracht und zwischen dem Grundkörper und dem Stumpf verteil werden.

## Patentansprüche

1. Verfahren zum Herstellen eines angepassten Liners (3) zur Anlage an einem Stumpf, (1) insbesondere Amputationsstumpf, mit folgendem Schritt:
- Auswählen eines vorgefertigten Liners (3) in Abhängigkeit von der Größe des Stumpfes (1);
**gekennzeichnet durch** die Schritte
- Aufbringen einer Füllmasse (2) auf Bereiche des Stumpfes (1), die von der Innenkontur des vorgefertigten Liners (3) abweichen;
- Verbinden der Füllmasse (2) mit dem vorgefertigten Liner (3).

2. Verfahren nach Anspruch 1, **dadurch gekennzeichnet, dass** der vorgefertigte Liner (3) nach dem Aufbringen der Füllmasse (2) auf den Stumpf (1) angelegt wird.

3. Verfahren nach Anspruch 1, **dadurch gekennzeichnet, dass** der vorgefertigte Liner (3) angelegt und die Füllmasse (2) nach dem Anlegen durch den vorgefertigten Liner (3) auf den Stumpf (1) aufgebracht wird.

4. Verfahren nach einem der voranstehenden Ansprüche, **dadurch gekennzeichnet, dass** die Füllmasse (2) im Bereich von Narbeneinzügen (11), schiefen Stumpfebenen, Dysmelien, Amelien oder konkaven Stellen aufgetragen wird.

5. Verfahren nach einem der voranstehenden Ansprüche, **dadurch gekennzeichnet, dass** der vorgefertigte Liner (3) aus einem Silikon-Werkstoff, Polyurethan-Werkstoff oder einem Copolymer-Werkstoff und die Füllmasse (2) aus unvernetztem Silikonkautschuk, Polyurethan oder Copolymer-Werkstoff ausgebildet sind.

6. Verfahren nach einem der voranstehenden Ansprüche, **dadurch gekennzeichnet, dass** vor dem Aufbringen der Füllmasse (2) die Hautoberfläche isoliert wird.

7. Verfahren nach einem der voranstehenden Ansprüche, **dadurch gekennzeichnet, dass** der vorgefertigte Liner (3) mit der Innenseite nach außen gekehrt und vom Stumpfende her auf den Stumpf (1) aufgerollt wird.

8. Verfahren nach einem der voranstehenden Ansprüche, **dadurch gekennzeichnet, dass** die Innenseite des vorgefertigten Liners (3) vor dem Anlegen entfettet wird.

9. Verfahren nach einem der voranstehenden Ansprüche, **dadurch gekennzeichnet, dass** die Füllmasse (2) sich mit dem vorgefertigten Liner (3) stoffschlüssig verbindet.

10. Verfahren nach einem der voranstehenden Ansprüche, **dadurch gekennzeichnet, dass** nach dem Verbinden der Füllmasse (2) mit dem vorgefertigten Liner (3) der Liner (3) abgenommen und mechanisch bearbeitet wird.

11. Verfahren nach einem der voranstehenden Ansprüche, **gekennzeichnet durch** das erneute Aufbringen von Füllmasse (2) und Verbinden mit dem zumindest teilweise angepassten Liner (3).

## Claims

1. Method for producing an adapted liner (3) for placement on a stump (1), particularly an amputation stump, comprising the following step:
- selecting a prefabricated liner (3) as a function of the size of the stump (1);
**characterized by** the steps
- applying a filling compound (2) to regions of the stump (1) that deviate from the inside contour of the prefabricated liner (3);
- connecting the filling compound (2) to the prefabricated liner (3).

2. Method according to Claim 1, **characterized in that** the prefabricated liner (3) is placed onto the stump (1) after the filling compound (2) has been applied.

3. Method according to Claim 1, **characterized in that** the prefabricated liner (3) is placed in position and the filling compound (2) is applied to the stump (1) through the prefabricated liner (3) after placement.

4. Method according to one of the preceding claims, **characterized in that** the filling compound (2) is applied in the region of scar contractions (11), oblique stump planes, dysmelias, amelias or concavities.

5. Method according to one of the preceding claims, **characterized in that** the prefabricated liner (3) is made from a silicone material, polyurethane material or copolymer material, and the filling compound (2) is made from uncrosslinked silicone rubber, polyurethane or copolymer material.

6. Method according to one of the preceding claims, **characterized in that** the skin surface is isolated before the filling compound (2) is applied.

7. Method according to one of the preceding claims, **characterized in that** the prefabricated liner (3), with the inner face turned outward, is rolled onto the stump (1) from the direction of the stump end.

8. Method according to one of the preceding claims, **characterized in that** the inner face of the prefabricated liner (3) is degreased before placement.

9. Method according to one of the preceding claims, **characterized in that** the filling compound (2) connects cohesively to the prefabricated liner (3).

10. Method according to one of the preceding claims, **characterized in that**, after the connection of the filling compound (2) to the prefabricated liner (3), the liner (3) is removed and mechanically worked.

11. Method according to one of the preceding claims, **characterized by** renewed application of filling compound (2) and connection to the at least partially adapted liner (3).

## Revendications

1. Procédé pour la réalisation d'un manchon ajusté (3) à rapporter sur un moignon (1), en particulier un moignon d'amputation, comprenant l'étape suivante consistant à :
- sélectionner un manchon préfabriqué (3) en fonction de la taille du moignon (1) ;
**caractérisé par** les étapes consistant à
- appliquer une masse de remplissage (2) sur des régions du moignon (1) qui s'écartent du contour intérieur du manchon préfabriqué (3) ;
- relier la masse de remplissage (2) avec le manchon préfabriqué (3).

2. Procédé selon la revendication 1, **caractérisé en ce que** le manchon préfabriqué (3) est rapporté sur le moignon (1) après l'application de la masse de remplissage (2).

3. Procédé selon la revendication 1, **caractérisé en ce que** le manchon préfabriqué (3) est rapporté et la masse de remplissage (2) est ensuite appliquée à travers le manchon préfabriqué (3) sur le moignon (1).

4. Procédé selon l'une des revendications précédentes, **caractérisé en ce que** la masse de remplissage (2) est appliquée dans la région de creux de cicatrice (11), de plans obliques du moignon, de dysmélies, d'amélies ou d'emplacements concaves.

5. Procédé selon l'une des revendications précédentes, **caractérisé en ce que** le manchon préfabriqué (3) est réalisé en un matériau à base de silicone, un matériau à base de polyuréthane ou un matériau à base de copolymère, et la masse de remplissage (2) est réalisée en caoutchouc au silicone non réticulé, en polyuréthane, ou en matériau à base de copolymère.

6. Procédé selon l'une des revendications précédentes, **caractérisé en ce que** la surface de la peau est isolée avant l'application de la masse de remplissage (2).

7. Procédé selon l'une des revendications précédentes, **caractérisé en ce que** le manchon préfabriqué (3) est retroussé avec le côté intérieur vers l'extérieur, et est enroulé sur le moignon (1) en partant depuis l'extrémité du moignon.

8. Procédé selon l'une des revendications précédentes, **caractérisé en ce que** la face intérieure du manchon préfabriqué (3) est débarrassée de graisse avant d'être rapporté.

9. Procédé selon l'une des revendications précédentes, **caractérisé en ce que** la masse de remplissage (2) établit une liaison par coopération de matières avec le manchon préfabriqué (3).

10. Procédé selon l'une des revendications précédentes, **caractérisé en ce qu'**après liaison de la masse de remplissage (2) avec le manchon préfabriqué (3), le manchon (3) est enlevé et est usiné de manière mécanique.

11. Procédé selon l'une des revendications précédentes, **caractérisé par** une nouvelle application de masse de remplissage (2) et une nouvelle liaison avec le manchon au moins partiellement ajusté (3).
